# EUROPEAN PATENT APPLICATION

(11) **EP 2 077 277 A1**
(43) Date of publication of application: **08.07.2009**
(21) Application number: 09005325.7
(22) Date of filing: 30.01.2003
(51) Int. Cl.: C07K 14/54, C07K 14/715, A61K 38/20, A61K 39/00, G01N 33/53

(54) **Use of mammalian cytokine; related reagents**

(30) Priority: 01.02.2002 US 353509 P
(62) Divisional of application: 03707610.6
(71) Applicant: Schering Corporation, Kenilworth, NJ 07033-0530 (US)
(72) Inventor: The designation of the inventor has not yet been filed
(74) Representative: Vossius & Partner

(57) **Abstract**

Provided are methods of modulating dendritic cell activity using agonists or antagonists of a mammalian cytokine. Also provided are methods of treating immune disorders.

SEQUENCE LISTING

<110>  Schering Corporation

<120>  Uses of mammalian cytokine; related reagents

<130>  DX01477K

<150>  60/353,509
<151>  2002-02-01

<160>  3

<170>  PatentIn version 3.1

<210>  1
<211>  159
<212>  PRT
<213>  Homo sapiens

<400>  1

Met Phe Pro Phe Ala Leu Leu Tyr Val Leu Ser Val Ser Phe Arg Lys
1               5                   10                  15

Ile Phe Ile Leu Gln Leu Val Gly Leu Val Leu Thr Tyr Asp Phe Thr
            20                  25                  30

Asn Cys Asp Phe Glu Lys Ile Lys Ala Ala Tyr Leu Ser Thr Ile Ser
        35                  40                  45

Lys Asp Leu Ile Thr Tyr Met Ser Gly Thr Lys Ser Thr Glu Phe Asn
    50                  55                  60

Asn Thr Val Ser Cys Ser Asn Arg Pro His Cys Leu Thr Glu Ile Gln
65                  70                  75                  80

Ser Leu Thr Phe Asn Pro Thr Ala Gly Cys Ala Ser Leu Ala Lys Glu
                85                  90                  95

Met Phe Ala Met Lys Thr Lys Ala Ala Leu Ala Ile Trp Cys Pro Gly
            100                 105                 110

Tyr Ser Glu Thr Gln Ile Asn Ala Thr Gln Ala Met Lys Lys Arg Arg
        115                 120                 125

Lys Arg Lys Val Thr Thr Asn Lys Cys Leu Glu Gln Val Ser Gln Leu
    130                 135                 140

Gln Gly Leu Trp Arg Arg Phe Asn Arg Pro Leu Leu Lys Gln Gln
145                 150                 155

<210>  2
<211>  459
<212>  PRT
<213>  Homo sapiens

<400>  2

<210> 3
<211> 371
<212> PRT
<213> Homo sapiens

<400> 3

Met Gly Arg Leu Val Leu Leu Trp Gly Ala Ala Val Phe Leu Leu Gly
1               5                   10                  15

Gly Trp Met Ala Leu Gly Gln Gly Gly Ala Ala Glu Gly Val Gln Ile
            20                  25                  30

Gln Ile Ile Tyr Phe Asn Leu Glu Thr Val Gln Val Thr Trp Asn Ala
        35                  40                  45

Ser Lys Tyr Ser Arg Thr Asn Leu Thr Phe His Tyr Arg Phe Asn Gly
    50                  55                  60

Asp Glu Ala Tyr Asp Gln Cys Thr Asn Tyr Leu Leu Gln Glu Gly His
65                  70                  75                  80

Thr Ser Gly Cys Leu Leu Asp Ala Glu Gln Arg Asp Asp Ile Leu Tyr
                85                  90                  95

Phe Ser Ile Arg Asn Gly Thr His Pro Val Phe Thr Ala Ser Arg Trp
            100                 105                 110

Met Val Tyr Tyr Leu Lys Pro Ser Ser Pro Lys His Val Arg Phe Ser
        115                 120                 125

Trp His Gln Asp Ala Val Thr Val Thr Cys Ser Asp Leu Ser Tyr Gly
    130                 135                 140

Asp Leu Leu Tyr Glu Val Gln Tyr Arg Ser Pro Phe Asp Thr Glu Trp
145                 150                 155                 160

Gln Ser Lys Gln Glu Asn Thr Cys Asn Val Thr Ile Glu Gly Leu Asp
                165                 170                 175

Ala Glu Lys Cys Tyr Ser Phe Trp Val Arg Val Lys Ala Met Glu Asp
            180                 185                 190

Val Tyr Gly Pro Asp Thr Tyr Pro Ser Asp Trp Ser Glu Val Thr Cys
      195                 200                 205

Trp Gln Arg Gly Glu Ile Arg Asp Ala Cys Ala Glu Thr Pro Thr Pro
    210                 215                 220

Pro Lys Pro Lys Leu Ser Lys Phe Ile Leu Ile Ser Ser Leu Ala Ile
225                 230                 235                 240

Leu Leu Met Val Ser Leu Leu Leu Leu Ser Leu Trp Lys Leu Trp Arg
                245                 250                 255

Val Lys Lys Phe Leu Ile Pro Ser Val Pro Asp Pro Lys Ser Ile Phe
            260                 265                 270

Pro Gly Leu Phe Glu Ile His Gln Gly Asn Phe Gln Glu Trp Ile Thr
        275                 280                 285

Asp Thr Gln Asn Val Ala His Leu His Lys Met Ala Gly Ala Glu Gln
    290                 295                 300

Glu Ser Gly Pro Glu Glu Pro Leu Val Val Gln Leu Ala Lys Thr Glu
305                 310                 315                 320

Ala Glu Ser Pro Arg Met Leu Asp Pro Gln Thr Glu Glu Lys Glu Ala
                325                 330                 335

Ser Gly Gly Ser Leu Gln Leu Pro His Gln Pro Leu Gln Gly Gly Asp
            340                 345                 350

Val Val Thr Ile Gly Gly Phe Thr Phe Val Met Asn Asp Arg Ser Tyr
        355                 360                 365

Val Ala Leu
    370

## Description

### FIELD OF THE INVENTION

The present invention relates generally to uses of mammalian cytokines. More specifically, the invention relates to identification of mammalian cytokine and inhibitors thereof that affect medical conditions such as allergy and inflammation.

### BACKGROUND OF THE INVENTION

For some time, it has been known that the mammalian immune response is based on a series of complex cellular interactions, called the "immune network". Recent research has provided new insights into the inner workings of this network. While it remains clear that much of the response does, in fact, revolve around the network-like interactions of lymphocytes, macrophages, granulocytes, and other cells, immunologists now generally hold the opinion that soluble proteins, known as cytokines, play a critical role in controlling these cellular interactions. Thus, there is considerable interest in the isolation, characterization, and mechanisms of action of cell modulatory factors, an understanding of which will lead to significant advancements in the diagnosis and therapy of numerous medical abnormalities, e.g., immune system disorders. Some of these factors are hematopoietic growth and/or differentiation factors, e.g., stem cell factor (SCF) and IL-7. See, e.g., Mire-Sluis and Thorpe (1998) Cytokines, Academic Press, San Diego, CA; Thomson (ed. 1998) The Cytokine Handbook, 3d ed., Academic Press, San Diego, CA; Metcalf and Nicola (1995) The Hematopoietic Colony Stimulating Factors, Cambridge Univ. Press; and Aggarwal and Gutterman (1991) Human Cytokines, Blackwell Publishing, Malden, MA.

Cytokines mediate cellular activities in a number of ways. Cytokines support the proliferation, growth, and differentiation of pluripotential hematopoietic stem cells into vast numbers of progenitors comprising diverse cellular lineages making up a complex immune system. Proper and balanced interactions between the cellular components are necessary for a healthy immune response. The different cellular lineages often respond in a different manner when cytokines are administered in conjunction with other agents.

Cytokines mediate communication between cells of the immune system, e.g., antigen presenting cells (APCs) and T lymphocytes. Dendritic cells (DCs) are the most potent of antigen presenting cells. See, e.g., Paul (ed.) (1993) Fundamental Immunology, 3d ed., Raven Press, NY. Antigen presentation refers to the cellular events in which a proteinaceous antigen is taken up, processed by antigen presenting cells (APC), and then recognized to initiate an immune response. The most active antigen presenting cells have been characterized as the macrophages (which are direct developmental products from monocytes), dendritic cells, and certain B cells. DCs are highly responsive to inflammatory stimuli such as bacterial lipopolysaccharides (LPS), and cytokines such as tumor necrosis factor alpha (TNFalpha). Cytokines or stimuli, such as LPS, can induce a series of phenotypic and functional changes in DC that are collectively referred to as maturation. See, e.g., Banchereau and Schmitt (eds.) (1995) Dendritic Cells in Fundamental and Clinical Immunology, Plenum Press, NY.

Dendritic cells can be classified as, e.g., interstitial dendritic cells of the heart, kidney, gut, and lung; Langerhans cells in the skin and mucous membranes; interdigitating dendritic cells in the thymic medulla and secondary lymphoid tissue; and blood and lymph dendritic cells. Although dendritic cells in each of these compartments are CD45⁺ leukocytes that apparently arise from bone marrow, they can exhibit differences that relate to maturation state and microenvironment. Maturational changes in DCs include, e.g., silencing of antigen uptake by endocytosis, upregulation of surface molecules related to T cell activation, and active production of a number of cytokines including TNFalpha and IL-12. Upon local accumulation of TNFalpha, DCs migrate to the T cell areas of secondary lymphoid organs to activate antigen specific T cells.

Cytokines and immune cells mediate specific physiological mechanisms or pathways, e.g., pathways leading to the various inflammatory disorders. About 20% of the population in Western countries suffers from inflammatory disorders, e.g., the allergic diseases, which include asthma, rhinitis, atopic dermatitis, and food allergy (see, e.g., A. B. Kay (2001) N. Engl. J. Med. 344:30-37). Allergic inflammation is the result of a complex immunological cascade leading to T cells to produce dysregulated TH2-derived cytokines such as IL-4, IL-5 and IL-13, where these cytokines trigger bronchial hyperreactvity, IgE production, eosinophilia, and mucus production (see, e.g., Busse and Lemanske, Jr. (2001) N. Engl. J. Med. 344:350-62; Holgate (2000) Br. Med. J. 320:231-234); and Renauld (2001) J. Clin. Pathol. 54:577-589).

Inflammation and immune reconstitution are two situations where it is desirable to use pharmaceutical or therapeutic intervention to modulate lymphocyte activity or proliferation, e.g., by modulating interactions between APCs and T cells. Inflammatory conditions dependent on APC-T cell interactions include, e.g., psoriasis, the allergies, and bronchial hypersensitivity. Immune reconstitution, the replenishment of the immune system, is useful in treating viral infections, e.g., HIV/AIDS, and in treating patients undergoing cytoablation, where cytoablation is effected, e.g., with radiation therapy or chemotherapy.

Psoriasis, an inflammatory disease of the skin, has a prevalence in Western countries of over 4% (Granstein (1996) J. Clin. Inv. 98:1695-1696; Christophers (2001) Clin. Exp. Dermatol. 26:314-320). The disease is subject to frequent relapses, is occasionally life-threatening, and is frequently associated with arthritis, i.e., psoriatic arthritis. T cells and keratinocytes are necessary for the development and persistence of psoriasis (Greaves and Weinstein (1995) New Engl. J. Med. 332:581-588; Robert and Kupper (1999) New Eng. J. Med. 341:1817-1828; Fearon and Veale (2001 Clin. Exp. Dermatol. 26:333-337). Dendritic cells and mast cells, for example, also contribute to psoriatic inflammation (Mrowietz, et al. (2001) Exp. Dermatol. 10:238-245; Ackermann, et al. (1999) Br. J. Dermatol. 140:624-633).

Bronchial hyperreactivity is the manifestation of pulmonary inflammatory diseases, including asthma, chronic obstructive pulmonary disease (COPD; chronic obstructive pulmonary disorder), chronic bronchitis, eosinophilic bronchitis, bronchiolitis, and viral bronchiolitis (Riffo-Vasquez and Spina (2002) Pharmacol. Therapeutics 94:185-211).

Asthma is a chronic disease characterized by increased bronchial responsiveness and by airway obstruction and inflammation. The disease accounts, e.g., for over 15% of pediatric emergencies (Crain, et al. (1995) Arch. Pediatr. Adolesc. Med. 149:893-901). APCs, T cells, B cells, eosinophils, mast cells, and basophils, contribute to the mechanism of asthma. APCs present antigen to T cells which, in turn, provoke B cells to produce IgE. Eosinophils, basophils, and mast cells release IL-4 which, in turn, promotes the differentiation of T cells into TH2 cells that secrete IL-4, IL-5, IL-10, and IL-13 after antigen stimulation. The IL-4 and IL-13, secreted by the TH2 cells and other cells, promotes activation of B cells (Marone (1998) Immunol. Today 19:5-9). B cells are stimulated to produce IgE by two types of signals, IL-4 or IL-13, and direct contact from T cells (Barnes and Lemanske (2001) New Engl. J. Med. 344:350-362). The released IgE activates mast cells which, in turn, cause constriction of the airways. Eosinophils produce major basic protein which directly damages the airways. IL-5 plays a central role in the development, survival, and recruitment of eosinophils (Barnes and Lemanske, supra).

COPD, which involves infiltration of bronchioles with lymphocytes, is the fourth leading cause of death in North America (Barnes (2000) New Engl. J. Med. 343:269-280). The disease is characterized by thickening of airway smooth muscle and inflammation of the airways, i.e., involving infiltration by monocytes, macrophages, CD4⁺ T cells, CD8⁺ T cells, and neutrophils in the lungs (Barnes (2000) Chest 117:10S-14S; Jeffery (1998) Thorax 53:129-136).

Immune reconstitution is a condition where modulation of lymphocyte proliferation is desirable. Immune reconstitution is accomplished, e.g., by bone marrow transplantation. Enhancing or stimulating T cell proliferation is desired in bone marrow transplantation following chemotherapy and in immune deficiency diseases, e.g., AIDS (Panteleo, et al. (1993) New Engl. J. Med. 328:327-335; Kovacs, et al. (1995) New Engl. J. Med. 332:567-575), as well as with use of therapeutic T cells, including genetically altered T cells (Terando and Chang (2002) Surg. Oncol. Clin. N. Am. 11:621-643; Gottschalk, et al. (2002) Adv. Cancer Res. 84:175-201). Immune reconstitution using bone marrow transplants or stem cell transplants is used following myeloablative and immunosuppressive therapy (Paloczi (2000) Immunol. Lett. 74:177-181; Ren-Heidenreich and Lum (2001) Curr. Gene Ther. 1:253-255).

Recipients of stem cell transplants experience delays in acquisition of fully functional lymphocytes, where these delays can extend beyond one year from the transplant. Naïve cells require a competent thymus for development. Hence, CD4⁺ T cell counts may be subnormal with bone marrow transplants, i.e., where the thymus has been damaged by radiotherapy (Novitzky and Davison (2001) Cytotherapy 3:211-220). Thus, stimulation of lymphocyte proliferation is a desirable goal because of the delays in T cell proliferation following bone marrow transplant, as well as in transplants where there the thymus is damaged.

Cytoablation followed by bone marrow transplant or stem cell therapy is used in the treatment of a number of autoimmune diseases, e.g., rheumatoid arthritis, systemic lupus erythematosus, Crohn's diseaes, and multiple sclerosis (Breedveld (2000) Arthritis Res. 2:268-269; McColl, et al. (1999) Ann. Intern. Med. 131:507-509; Laar (2000) Arthritis Res. 2:270-275), as well as in treatment of cancers such as non-Hodgkin's lymphoma and leukemia (Kay, et al. (2002) Hematology (Am. Soc. Hematol. Educ. Program) 193-213; Hagemeister (2002) Cancer Chemother. Pharmocol. 49 Suppl. 1:S13-20). Thus, there is an increased need for stimulating T cell proliferation after cytoablation.

Graft-versus-host disease (GVHD) is a problem with bone marrow transplants. GVHD is a consequence of allogeneic transplants, where GVHD can be prevented by ex vivo depletion of the T cells in the graft (Andre-Schmutz, et al. (2002) Lancet 360:130-137; Aversa, et al. (1998) New Engl. J. Med. 339:1186-1193). Ex vivo treatment of lymphocytes, e.g., by treatment with cytokines or nucleic acids, followed by introduction into a subject is described. See, e.g., Ernerudh, et al. (2002) Curr. Med. Chem. 9:1497-1505; Cavazzana-Calvo, et al. (2002) Semin. Hematol. 39:32-40; Gunzer and Grabbe (2001) Crit. Rev. Immuol. 21:133-145; Gokmen, et al. (2001) J. Hematother. Stem Cell Res. 10:53-66. The above-described ex vivo depletion ofT cells, however, exacerbates the T cell deficiency. Hence, there is an increased need for stimulating T cell proliferation to promote immune reconstitution, where T cells were depleted ex vivo, prior to the graft.

Currently, there is an interest in using hematopoietic growth factors and cytokines to stimulate T cell proliferation following bone marrow transplants (Symann, et al. (1989) Cancer Treat. Rev. 16 Suppl. A:15-19; Lenarsky (1993) Am. J. Pediatr. Hematol. Oncol. 15:49-55). A problem with current methods is skewing the T cell repertoire to oligoclonality (Marktel, et al. (2002) Blood, October 3, 2002, epub ahead of print). Hence, there is a need to stimulate T cell proliferation by methods that maintain polyclonality.

The invention provides methods for modulating dendritic cells (DCs) for the treatment of inflammatory conditions dependent on APC/T cell interactions, and for effecting immune reconstitution. Dendritic cells, the professional antigen presenting cells, play a role in stimulating T cell activation and prolilferation. DCs, the professional antigen presenting cells, play an important role in the pathogenesis of allergic diseases. See, e.g., Banchereau and Steinman (1998) Nature 392:245-252; Stumbles (1999) Immunol. Cell Biol. 77:428-433; Lambrecht (2001) Clin. Exp. Allergy 31, 206-218; Semper et al. (1995) Adv. Exp. Med. Biol. 378:135-138. However, the initial signal that primes DCs to induce T cells producing pro-allergic TH2 cytokines is unknown (see, e.g., D. von Bubnoff, et al. (2001) J. Allergy Clin. Immunol. 108:329-339). Although skin keratinocytes and mucosal epithelial cells were shown to produce pro-inflammatory cytokines such as IL-1, IL-6, IL-8, GM-CSF and TNFalpha following activation (S. Nozaki, et al. (1992) Adv. Dermatol. 7:83-100; and discussion 101; T. S. Kupper (1990) J. Invest. Dermatol. 94:146S-150S; P. F. Piguet (1992) Sprinter Semin. Immunopathol. 13:345-354; and 1. R. Williams and T. S. Kupper (1996) Life Sci. 58:1485-1507), none of these cytokines can explain the mechanism underlying the induction of allergic inflammation (See, e.g. D. von Bubnoff, supra).

Thymic stromal lymphopoietin (hTSLP/IL-50) (SEQ ID NO:1) is a novel IL-7-like cytokine, cloned from a murine thymic stromal cell line (see, e.g., J. E. Sims et al., (2000) J. Exp. Med. 192:671-680; and USSN 09/963,347, filed September 24, 2001). The mature coding region of human TSLP is amino acids 29-159 (Reche, et al. (2001) J Immunol. 167:336-343). The TLSP/IL-50-receptor is a heterodimer, consisting of the IL-7R-alpha chain (SEQ ID NO:2) and a common gamma-like receptor chain (TSLP receptor; TSLPR) (SEQ ID NO:3) (see, e.g., Tonozuka et al. (2001) Cytogenet. Cell Genet. 93:23-25; Pandey et al. (2000) Nat. Immunol. 1:59-64; L. S. Park et al., (2000) J. Exp. Med. 192:659-670; and Reche et al., supra. While mouse TSLP/IL-50 (SEQ ID NO:1) supports murine early B and T cell developments (see, e.g. Levin et al. (1999) J. Immunol. 162:677-683; Ray, et al. (1996) Eur. J. Immunol. 26:10-16), hTSLP/IL-50 (SEQ ID NO:1) activates CD11c⁺DCs, but do not have any direct biological effects on B cells, T cells, NK cells, neutrophils, nor mast cells (see, e.g., Reche, et al., supra). This is in accordance with the co-expression of mRNA for hTSLP/IL-50 receptor delta2 subunit and the IL-7R-alpha chain in CD11c⁺ DCs, but not in other cell types.

The mechanisms and pathogenesis of inflammation, in particular, allergic inflammation, are not fully understood, and as such several therapies are as yet unknown. The present invention provides evidence that hTSLP/IL-50 (SEQ ID NO:1) can mediate various inflammatory disorders by its action on certain subsets of immune cells, in particular, dendritic cells.

### SUMMARY OF THE INVENTION

The present invention is based, in part, upon the discovery of the effect of hTSLP/IL-50 (SEQ ID NO:1) on antigen presenting cell, e.g., dendritic cells (DC), activity, in particular, DC priming of T cells resulting in inflammation, e.g., psoriasis or allergic inflammation.

The invention provides a method of modulating antigen presenting cell (APC) priming of a T cell comprising contacting the APC with an agonist of TSLP/IL-50 (SEQ ID NO:1) or TSLP/IL-50 receptor (TSLP/IL-50R) (SEQ ID NOs:2, 3); or an antagonist of TSLP/IL-50 (SEQ ID NO:1) or TSLP/IL-50R (SEQ ID NOs:2, 3). Also provided is the above method, wherein the T cell is a naïve CD4⁺ T cell, a central memory T cell, or an effector memory T cell; wherein the APC is a CD11c⁺ dendritic cell (DC); wherein the priming stimulates the proliferation of the T cell; wherein the proliferation is polyclonal; or
wherein the interaction between the APC and the T cell is autologous or allogeneic, or
wherein the interaction is autologous and yields a central memory T cell phenotype.

Further provided is the above method wherein the agonist or antagonist comprises a humanized antibody; a monoclonal antibody; a polyclonal antibody; an Fab fragment; an F(ab')₂ fragment; or a peptide mimetic of an antibody; or wherein the agonist is TSLP/IL-50 (SEQ ID NO:1), or an antigenic fragment thereof.

In another embodiment, the invention encompasses a method of treating a subject suffering from an immune disorder comprising treating with or administering an effective amount of an agonist of TSLP/IL-50 (SEQ ID NO:1) or TSLP/IL-50 R (SEQ ID NOs:2,3); or an antagonist of TSLP/IL-50 (SEQ ID NO:1) or TSLP/IL-50R (SEQ ID NOs:2, 3). Also encompassed is the above method, wherein the immune disorder is an inflammatory condition and the administration comprises an effective amount of an antagonist of TSLP/IL-50 (SEQ ID NO:1) or TSLP/IL-50R (SEQ ID NOs:2, 3); wherein the immune disorder is psoriasis, psoriatic arthritis, or pulmonary inflammatory response; or wherein the pulmonary inflammatory disease is asthma or chronic obstructive pulmonary disorder (COPD). Further provided is the above method, wherein the immune disorder is immunodeficiency and the administration comprises an effective amount of an agonist of TSLP/IL50 (SEQ ID NO:1);
wherein the immunodeficiency is a result of cytoablation or viral infection causing immunosuppression; wherein the administration comprises ex vivo treatment of autologous or allogeneic antigen presenting cells (APCs); or wherein the administration comprises ex vivo treatment of APCs with an effective amount of an agonist of TSLP/IL50 (SEQ ID NO:1). The invention also contemplates the above method, wherein the agonist or antagonist comprises a humanized antibody; a monoclonal antibody; a polyclonal antibody; an Fab fragment; an F(ab')₂ fragment; or a peptide mimetic of an antibody; or wherein the agonist is TSLP/IL-50 (SEQ ID NO:1), or an antigenic fragment thereof.

Further contemplated is a method of inducing production ofIL-4, IL-5, and IL-13 by a T cell comprising contacting an APC with an agonist of TSLP/IL-50 or TSLP/IL-50 receptor, and priming the T cell with the APC.

The invention also encompasses a method of modulating TH2 response in a subject comprising administration of an agonist of TSLP/IL-50 (SEQ ID NO:1) or TSLP/IL-50 receptor (TSLP/IL-50R) (SEQ ID NOs:2, 3); or an antagonist of TSLP/IL-50 (SEQ ID NO:1) or TSLP/IL-50R (SEQ ID NOs:2, 3).

### DETAILED DESCRIPTION OF THE PREFERRED EMBODIMENTS

As used herein, including the appended claims, the singular forms of words such as "a," "an," and "the," include their corresponding plural references unless the context clearly dictates otherwise.

All references cited herein are incorporated herein by reference to the same extent as if each individual publication or patent application was specifically and individually indicated to be incorporated by reference.
I.Definitions.
   "Activation," "stimulation," and "treatment," as it applies to cells or to receptors, may have the same meaning, e.g., activation, stimulation, or treatment of dendritic cells (DC) with a ligand, unless indicated otherwise by the context or explicitly.
   "Administration" and "treatment," as it applies to treatment of a human subject or animal, refers to contact of a pharmaceutical, therapeutic, or diagnostic agent or composition to the subject or animal. "Administration" and "treatment"also means ex vivo treatment to, e.g., a cell, tissue, or organ, followed by contact of the cell, tissue, or organ, to the subject or animal, even where the agent or composition has been metabolized, altered, or degraded, during the ex vivo treatment.
   "Allogeneic," as it applies to cells or to a reaction between cells, refers, e.g., to an interaction where the major histocompatibility complex (MHC) of a first cell is recognized as foreign by a second cell. "Autologous," as it applies to cells or to a reaction between cells, refers, e.g., to an interaction where the MHC of a first cell is recognized as self by a second cell (Abbas, et al. (2000) Cellular and Molecular Immunology, 4th ed., W.B. Saunders Co., Philadelphia).
   "Effective amount" means an amount sufficient to ameliorate a symptom or sign of the medical condition.
   "Polyclonal" expansion or proliferation means that proliferation of a cell involves maintenance of the phenotype, while "oligoclonal" expansion or proliferation means that the phenotype is altered (Duarte, et al. (2002) Gene Therapy 9:1359-1368).
   "Sensitivity," e.g., sensitivity of T cell receptor (TCR), means that binding of a ligand to TCR results in a detectable change in the TCR, or in events or molecules specifically associated with the TCR, e.g., TCR conformational change or phosphorylation, change in proteins associated with the TCR, or change in TCR-associated genetic expression.
II.General.
   hTSLP/IL-50 (SEQ ID NO:1) (a.k.a. Thymic Stromal Lymphopoietin; TSLP) was originally discovered in the mouse and found to play a similar role as its homologue IL-7 in supporting early B and T cell development (see, e.g., Sims, supra; Levin et al., supra; and Ray, et al., supra). Mouse TSLP/IL-50 (SEQ ID NO:1) did not activate mouse DCs isolated from spleen, or generated from monocytes or bone marrow. The present invention demonstrates that human TSLP/IL-50 (SEQ ID NO:1) is a novel DC activator. hTSLP/IL-50 (SEQ ID NO:1) displays several unique features, when compared with other DC activation factors, e.g., CD40-ligand, LPS, or IL-7. For example, it induces the highest levels of CD40 and CD80 on DCs; it activates DCs to induce the most potent naïve CD4 T cell proliferation and expansion; it does not appear to induce DCs to produce several of the known proinflammatory cytokines, but rather it induces the production of TH2 attracting chemokines TARC and MDC; and it causes DCs to prime naïve CD4⁺ T cells to produce high levels of the TH2 cytokines IL-4, IL-5, IL-13, and TNFalpha. Interestingly, production of the anti-inflammatory cytokine IL-10 and TH1 cytokine IFN-gamma are inhibited. These features strongly suggest that hTSLP/IL-50 (SEQ ID NO:1) represent a critical mediator in uncontrolled inflammation, in particular, allergic inflammation.
   Activation of DCs appears to be a critical step in the pathogenesis of TH2-mediated allergic inflammations, e.g., asthma. Dendritic cells presenting allergen to Th2 cells activate the Th2 cells to release cytokines, e.g., IL-4, IL-5, and IL-13, where these cytokines contribute in differing ways to the pathology of asthma. IL-4 stimulates increases in airway endothelial cell adhesion molecules and chemokine production, IL-5 provokes eosinophil production, while IL-13 promote smooth muscle hyperreactivity (Lewis (2002) Curr. Opinion Immunol. 14:644-651). The IL-4 stimulated cell adhesion molecules serve as receptors for inflammatory cells (Striz, et al. (1999) Am J. Physiol. 277:L58-L64). IL-4 and IL-13 activate B cells, resulting in B cell proliferation and synthesis of IgE (Busse and Lemanske (2001 New Engl. J. Med. 344:350-362). IL-4 is overexpressed in airways of allergic asthmatics, while IL-13 is overexpressed in airways in both allergic and non-allergic asthma (Wills-Karp, et al. (1998) Science 282:2258-2260). IL-4 seems more important in primary allergen sensitization, while IL-13 appears more important during secondary exposure to allergen (Kips (2001) Eur. Resp. J. Suppl. 34:24s-33s).
   Although DCs from allergic individuals preferentially induce a TH2-type response with (see, e.g.,Hammad et al., (2001) Blood 98, 1135-41) or without (see, e.g., P. A. Stumbles, supra; McWilliam et al. (1996) J. Exp. Med. 184:2429-32; N. Novak et al. (1999) Allergy 54:792-803; Tunon-De-Lara et al. (1996) Clin. Exp. Allergy 26:648-655; and Holt (1997) Adv. Exp. Med. Biol. 417:301-306) priming with an allergen, the molecular mechanism underlying the signaling of DCs to induce TH2 allergic diseases is not clearly understood. The present findings that hTSLP/IL-50 (SEQ ID NO:1) is highly expressed by keratinocytes of atopic dermatitis and hTSLP/IL-50 (SEQ ID NO:1)-activated DCs strongly prime naive CD4⁺ T cells to produce IL-4, IL-5, IL-13 and TNFalpha, suggest that hTSLP/IL-50 (SEQ ID NO:1) represents the missing critical factor in understanding the pathogenesis of allergic diseases. hTSLP/IL-50 (SEQ ID NO:1) produced by epithelial cells, or other stromal cells at the site of antigen entry, will activate DCs and stimulate DCs to produce TH2-attracting chemokines such as TARC and MDC. hTSLP/IL-50 (SEQ ID NO:1)-activated DCs migrate into the draining lymph nodes to induce allergen-specific T cell proliferation and differentiation into TH2 cells. These allergen-specific TH2 T cells may migrate back towards TARC and MDC within the original site of inflammation, to trigger allergic inflammation, thus establishing a direct functional link between epithelial cells, DCs and T cell-mediated immune responses.
   Unlike classical TH2 cells which produce IL-4, IL-5, IL10 and IL-13, human CD4⁺ T cells activated by hTSLP/IL-50 stimulated-DCs produce IL-4, IL-5 and IL-13, but not IL-10. Although IL-10 has been historically included as a TH2 cytokine(see, e.g., Abbas, et al. (1996) Nature 383:787-793), its contribution to the TH2-mediated allergic inflammation has been controversial. Whereas some studies showed that IL-10 mRNA levels in lung, gut and skin were increased in patients with allergic asthma or atopic dermatitis (see, e.g., Robinson et al. (1996) Am. J. Respir. Cell Mol. Biol. 14:113-117), direct measurement of IL-10 protein by ELISA (Enzyme-Linked Immunosorbent Assay) showed a markedly lower IL-10 levels in the bronchoalveolar lavage or in the culture supernatants of activated peripheral blood mononuclear cells from atopic patients, compared with normal control subjects (see, e.g., Borish et al. (1996) J. Allergy Clin. Immunol. 97:1288-96). Studies in mouse models confirm a role of IL-10 in suppressing airway inflammation and cytokine production (see, e.g., Akbari,et al. (2001) Nat. Immunol. 2:725-731; and Zuany-Amorim et al. (1995) J. Clin. Invest. 95:2644-2651). Therefore, high levels of IL-4, IL-5, IL-13 and TNFalpha, and decreased levels of IL-10 and IFN-gamma produced by hTSLP/IL-50 stimulated-DC activated T cells, may represent the real allergic inflammatory cytokines underlying the pathophysiology of atopic dermatitis or asthma. IL-10 is an anti-inflammatory cytokine, but not a pro-allergic TH2 cytokine.
   Further described is the first evidence that epithelial cells of skin and mucosa directly interact with DCs during allergic inflammation by producing TSLP/IL-50 (SEQ ID NO:1). hTSLP/IL-50 (SEQ ID NO:1) not only potently activates DCs, but also endorse DCs with the ability to polarize naive T cells to produce pro-allergic TH2 cytokines. hTSLP/IL-50 (SEQ ID NO:1) represents a novel target to block inflammatory and allergic diseases.
   The present invention provides methods and reagents to enhance the TH2-mediated response by agonizing the activities of TSLP/IL-50 (SEQ ID NO:1). Enhance of this response is useful in the treatment of disorders due to suppression of the immune system, e.g., HIV. Augmentation of dendritic cell activity will be useful in the treatment of viral, bacterial, or fungal infections. TSLP/IL-50 (SEQ ID NO:1) and/or agonists thereof will also be useful as vaccine adjuvants.
   Suppression of DC response is useful for the treatment of several immune disorders and condition, e.g., allergic inflammation, bronchial hyperreactivity, asthma, rhinitis, food allergy, transplant rejection, graft-vs-liost disease, autoimmune diseases, viral infections that cause immunosuppression, psoriasis, and atopic dermatitis.
III. Antagonists and agonists.
   Blockage of the activities of hTSLP/IL-50 (SEQ ID NO:1) can be achieved by antagonists of the cytokine, e.g., antibodies to the ligand, antibodies to the receptor, etc. Interference with the ligand-receptor interaction has proven to be an effective strategy for the development of antagonists.
   There are various means to antagonize the activity mediated by ligand. Two apparent means are to block the ligand with antibodies; a second is to block the receptor with antibodies. Various epitopes will exist on each which will block their interaction, e.g., causing steric hindrance blocking interaction. The correlation of ability to block signaling would not necessarily be expected to correlate with binding affinity to either ligand or receptors. Another means is to use a ligand mutein which retains receptor binding activity, but fails to induce receptor signaling. The mutein may be a competitive inhibitor of signaling ligand.
   Alternatively, small molecule libraries may be screened for compounds which may block the interaction or signaling mediated by an identified ligand-receptor pairing.
   The present invention provides for the use of an antibody or binding composition which specifically binds to a specified cytokine ligand, preferably mammalian, e.g., primate, human, cat, dog, rat, or mouse. Antibodies can be raised to various cytokine proteins, including individual, polymorphic, allelic, strain, or species variants, and fragments thereof, both in their naturally occurring (full-length) forms or in their recombinant forms. Additionally, antibodies can be raised to receptor proteins in both their native (or active) forms or in their inactive, e.g., denatured, forms. Anti-idiotypic antibodies may also be used.
   A number of immunogens may be selected to produce antibodies specifically reactive with ligand or receptor proteins. Recombinant protein is a preferred immunogen for the production of monoclonal or polyclonal antibodies. Naturally occurring protein, from appropriate sources, e.g., primate, rodent, etc., may also be used either in pure or impure form. Synthetic peptides, made using the appropriate protein sequences, may also be used as an immunogen for the production of antibodies. Recombinant protein can be expressed and purified in eukaryotic or prokaryotic cells as described, e.g., in Coligan, et al. (eds. 1995 and periodic supplements) Current Protocols in Protein Science, John Wiley and Sons, New York, NY; and Ausubel, et al. (eds. 1987 and periodic supplements) Current Protocols in Molecular Biology, Greene/Wiley, New York, NY. Naturally folded or denatured material can be used, as appropriate, for producing antibodies. Either monoclonal or polyclonal antibodies may be generated, e.g., for subsequent use in immunoassays to measure the protein, or for immunopurification methods.
   Methods of producing polyclonal antibodies are well known to those of skill in the art. Typically, an immunogen, preferably a purified protein, is mixed with an adjuvant and animals are immunized with the mixture. The animal's immune response to the immunogen preparation is monitored by taking test bleeds and determining the titer of reactivity to the protein of interest. For example, when appropriately high titers of antibody to the immunogen are obtained, usually after repeated immunizations, blood is collected from the animal and antisera are prepared. Further fractionation of the antisera to enrich for antibodies reactive to the protein can be performed if desired. See, e.g., Harlow and Lane; or Coligan. Immunization can also be performed through other methods, e.g., DNA vector immunization. See, e.g., Wang, et al. (1997) Virology 228:278-284.
   Monoclonal antibodies may be obtained by various techniques familiar to researchers skilled in the art. Typically, spleen cells from an animal immunized with a desired antigen are immortalized, commonly by fusion with a myeloma cell. See, Kohler and Milstein (1976) Eur. J. Immunol. 6:511-519. Alternative methods of immortalization include transformation with Epstein Barr Virus, oncogenes, or retroviruses, or other methods known in the art. See, e.g., Doyle, et al. (eds. 1994 and periodic supplements) Cell and Tissue Culture: Laboratory Procedures, John Wiley and Sons, New York, NY. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host. Alternatively, one may isolate DNA sequences which encode a monoclonal antibody or a binding fragment thereof by screening a DNA library from human B cells according, e.g., to the general protocol outlined by Huse, et al. (1989) Science 246:1275-1281.
   Antibodies or binding compositions, including binding fragments, single chain antibodies, Fv, Fab, or F(ab')₂ fragments of antibodies, against predetermined fragments of ligand or receptor proteins can be raised by immunization of animals with conjugates of the fragments of the ligand or receptor proteins with carrier proteins. Monoclonal antibodies are prepared from cells secreting the desired antibody. These antibodies can be screened for binding to normal or defective protein. These monoclonal antibodies will usually bind with at least a K_{D} of about 1 mM, more usually at least about 300 µM, typically at least about 10 µM, more typically at least about 30 µM, preferably at least about 10 µM, and more preferably at least about 3 µM or better.
   In some instances, it is desirable to prepare monoclonal antibodies (mAbs) from various mammalian hosts, such as mice, rodents, primates, humans, etc. Description of techniques for preparing such monoclonal antibodies may be found in, e.g., Stites, et al. (eds.) Basic and Clinical Immunology, 4th ed., Lange Medical Publications, Los Altos, CA, and references cited therein; Harlow and Lane (1988) Antibodies: A Laboratory Manual CSH Press; Goding (1986) Monoclonal Antibodies: Principles and Practice, 2nd ed., Academic Press, New York, NY; and particularly in Kohler and Milstein (1975) Nature 256:495-497, which discusses one method of generating monoclonal antibodies. Summarized briefly, this method involves injecting an animal with an immunogen. The animal is then sacrificed and cells taken from its spleen, which are then fused with myeloma cells. The result is a hybrid cell or "hybridoma" that is capable of reproducing in vitro. The population of hybridomas is then screened to isolate individual clones, each of which secrete a single antibody species to the immunogen. In this manner, the individual antibody species obtained are the products of immortalized and cloned single B cells from the immune animal generated in response to a specific site recognized on the immunogenic substance.
   Other suitable techniques involve selection of libraries of antibodies in phage or similar vectors. See, e.g., Huse, et al. (1989) Science 246:1275-1281; and Ward, et al. (1989) Nature 341:544-546. The polypeptides and antibodies of the present invention may be used with or without modification, including chimeric or humanized antibodies. Frequently, the polypeptides and antibodies will be labeled by joining, either covalently or non-covalently, a substance which provides for a detectable signal. A wide variety of labels and conjugation techniques are known and are reported extensively in both the scientific and patent literature. Suitable labels include radionuclides, enzymes, substrates, cofactors, inhibitors, fluorescent moieties, chemiluminescent moieties, magnetic particles, and the like. Patents teaching the use of such labels include U.S. Pat. Nos. 3,817,837; 3,850,752; 3,939,350; 3,996,345; 4,277,437; 4,275,149; and 4,366,241. Also, recombinant immunoglobulins may be produced, see, Cabilly, U.S. Pat. No. 4,816,567; and Queen, et al. (1989) Proc. Nat'l Acad. Sci. USA 86:10029-10033; or made in transgenic mice, see Mendez, et al. (1997) Nature Genetics 15:146-156; also see Abgenix and Medarex technologies.
   Antibodies are merely one form of specific binding compositions. Other binding compositions, which will often have similar uses, include molecules that bind with specificity to ligand or receptor, e.g., in a binding partner-binding partner fashion, an antibody-antigen interaction, or in a natural physiologically relevant protein-protein interaction, either covalent or non-covalent, e.g., proteins which specifically associate with desired protein. The molecule may be a polymer, or chemical reagent. A functional analog may be a protein with structural modifications, or may be a structurally unrelated molecule, e.g., which has a molecular shape which interacts with the appropriate binding determinants. Antibody binding compounds, including binding fragments, of this invention can have significant diagnostic or therapeutic value. They can be useful as non-neutralizing binding compounds and can be coupled to toxins or radionuclides so that when the binding compound binds to the antigen, a cell expressing it, e.g., on its surface, is killed. Further, these binding compounds can be conjugated to drugs or other therapeutic agents, either directly or indirectly by means of a linker, and may effect drug targeting.
   Antibodies to TSLP/IL-50 (SEQ ID NO:1) are available (Soumelis, et al., supra). Regions of increased antigenicity in human TSLP/IL-B50 (SEQ ID NO:1) include KAAYL (amino acids 40-44); KD (49-50); KS (59-60); PHC (73-75); ASLAK (91-95); TKAAL (102-106); KKRRKRKV (125-132); and PLLKQ (154-158). Antibodies against IL-7Ralpha (SEQ ID NO:2) are available (Pandey, et al., supra). Anti-TSLPR antibodies are available (R & D Systems, Minneapolis, MN, cat. no. MAB981; DNAX Research, Inc., Palo Alto, CA). Antibodies are also prepared against TSLPR (SEQ ID NO:3) by immunization with, e.g., regions of increased antigenicity determined by the Welling plot of Vector NTI® Suite (Informax, Inc, Bethesda, MD). Regions of increased antigenicity in human TSLPR include HYR (amino acid residues 59-61); YYLKP (115-119); KHV (123-125); WHQDAV (129-134); KPKLSK (226-231); and AHLHKM (294-299) from SEQ ID NO:3, where the N-terminal region is cytosolic and the transmembrane region of human TSLPR is predicted to occur at about residues 203-207 (Blagoev, et al. (2002) Gene 284:161-168; Park, et al., supra).
   Agonists include the TSLP/IL-50 (SEQ ID NO:1) cytokine protein itself, which can be used to induce receptor signaling.
IV. Diagnostic uses; therapeutic compositions, methods.

The invention provides means to address various inflammation related disorders, e.g., allergic inflammation. The etiology and pathogenesis are often not well understood, but they cause significant discomfort or morbidity in patients. As noted below, administration of TSLP/IL-50 (SEQ ID NO:1) to CD11c⁺ DCs results in the priming of naïve CD4⁺ T cells to produce IL-4, IL-5, IL-13, and TNFalpha, and thus agonists or antagonists may offer a therapeutic modality to enhance or suppress the immune system.

Diagnostic methods include such aspects as prediction of prognosis; definition of subsets of patients who will either respond or not respond to a particular therapeutic course; diagnosis of bone or immune related disorders or subtypes of these disorders; or assessing response to therapy. The invention contemplates an antibody, or binding fragment thereof, comprising a detectable label, e.g., a fluorescent, epitopic, enzymatically active, or radioactive label.

Antagonists or agonists to TSLP/IL-50 (SEQ ID NO:1) activity can be implicated in a manner suggesting significant therapeutic effects, e.g., to decrease or prevent occurrence of symptoms. The antagonists and/or agonists of the present invention can be administered alone or in combination with another inhibitor or agonist of the same or accompanying pathway; or other compounds used for the treatment of symptoms, e.g., antagonists, or steroids such as glucocorticoids.

This may be effected by either direct administration of the agonist or antagonist, or perhaps using a gene therapy strategy. Antagonism may be effected, e.g., by antisense treatment, antibodies, or other suppression of TSLP/IL-50 (SEQ ID NO:1) effects.

To prepare pharmaceutical or sterile compositions including the antibody, binding composition thereof, cytokine agonist, or small molecule antagonist, the entity is admixed with a pharmaceutically acceptable carrier or excipient which is preferably inert. Preparation of such pharmaceutical compositions is known in the art, see, e.g., Remington's Pharmaceutical Sciences and U.S. Pharmacopeia: National Formulary, Mack Publishing Company, Easton, PA (1984).

Antibodies, binding compositions, or cytokines are normally administered parentally, preferably intravenously. Since such proteins or peptides may be immunogenic they are preferably administered slowly, either by a conventional i.v. administration set or from a subcutaneous depot, e.g. as taught by Tomasi, et al, U.S. Pat. No. 4,732,863. Means to minimize immunological reactions may be applied. Small molecule entities may be orally active.

When administered parenterally the biologics will be formulated in a unit dosage injectable form (solution, suspension, emulsion) in association with a pharmaceutically acceptable parenteral vehicle. Such vehicles are typically inherently nontoxic and nontherapeutic. The therapeutic may be administered in aqueous vehicles such as water, saline, or buffered vehicles with or without various additives and/or diluting agents. Alternatively, a suspension, such as a zinc suspension, can be prepared to include the peptide. Such a suspension can be useful for subcutaneous (SQ) or intramuscular (IM) injection. The proportion of biologic and additive can be varied over a broad range so long as both are present in effective amounts. The antibody is preferably formulated in purified form substantially free of aggregates, other proteins, endotoxins, and the like, at concentrations of about 5 to 30 mg/ml, preferably 10 to 20 mg/ml. Preferably, the endotoxin levels are less than 2.5 EU/ml. See, e.g., Avis, et al. (eds.)(1993) Pharmaceutical Dosage Forms: Parenteral Medications, 2nd ed., Dekker, NY; Lieberman, et al. (eds. 1990) Pharmaceutical Dosage Forms: Tablets 2nd ed., Dekker, NY; Lieberman, et al. (eds. 1990) Pharmaceutical Dosage Forms: Disperse Systems, Dekker, NY).

Selecting an administration regimen for a therapeutic depends on several factors, including the serum or tissue turnover rate of the entity, the level of symptoms, the immunogenicity of the entity, and the accessibility of the target cells, timing of administration, etc. Preferably, an administration regimen maximizes the amount of therapeutic delivered to the patient consistent with an acceptable level of side effects. Accordingly, the amount of biologic delivered depends in part on the particular entity and the severity of the condition being treated. Guidance in selecting appropriate antibody doses is found in, e.g. Bach et al., chapter 22, in Ferrone, et al. (eds.) (1985) Handbook of Monoclonal Antibodies, Noges Publications, Park Ridge, NJ; and Haber, et al. (eds.) (1977) Antibodies in Human Diagnosis and Therapy, Raven Press, New York, NY (Russell, pgs. 303-357, and Smith, et al., pgs. 365-389). Alternatively, doses of cytokine or small molecules are determined using standard methodologies.

Determination of the appropriate dose is made by the clinician, e.g., using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, e.g., the inflammation or level of inflammatory cytokines produced. Preferably, a biologic that will be used is derived from the same species as the animal targeted for treatment, thereby minimizing a humoral response to the reagent.

The total weekly dose ranges for antibodies or fragments thereof, which specifically bind to ligand or receptor range generally from about 10 µg, more generally from about 100 µg, typically from about 500 µg, more typically from about 1000 µg, preferably from about 5 mg, and more preferably from about 10 mg per kilogram body weight. Generally the range will be less than 100 mg, preferably less than about 50 mg, and more preferably less than about 25 mg per kilogram body weight. Agonist or small molecule therapeutics may be used at similar molarities.

The weekly dose ranges for antagonists of cytokine receptor mediated signaling, e.g., antibody or binding fragments, range from about 1 µg, preferably at least about 5 µg, and more preferably at least about 10 µg per kilogram of body weight. Generally, the range will be less than about 1000 µg, preferably less than about 500 µg, and more preferably less than about 100 µg per kilogram of body weight. Dosages are on a schedule which effects the desired treatment and can be periodic over shorter or longer term. In general, ranges will be from at least about 10 µg to about 50 mg, preferably about 100 µg to about 10 mg per kilogram body weight. Cytokine agonists or small molecule therapeutics will typically be used at similar molar amounts, but because they likely have smaller molecular weighs, will have lesser weight doses.

The present invention also provides for administration of biologics in combination with known therapies, e.g., steroids, particularly glucocorticoids, which alleviate the symptoms, e.g., associated with inflammation, or antibiotics or anti-infectives. Daily dosages for glucocorticoids will range from at least about 1 mg, generally at least about 2 mg, and preferably at least about 5 mg per day. Generally, the dosage will be less than about 100 mg, typically less than about 50 mg, preferably less than about 20 mg, and more preferably at least about 10 mg per day. In general, the ranges will be from at least about 1 mg to about 100 mg, preferably from about 2 mg to 50 mg per day. Suitable dose combinations with antibiotics, anti-infectives, or anti-inflammatories are also known.

Typical mammalian hosts will include mice, rats, cats, dogs, and primates, including humans. An effective amount for a particular patient may vary depending on factors such as the condition being treated, the overall health of the patient, the method route and dose of administration and the severity of side affects. When in combination, an effective amount is in ratio to a combination of components and the effect is not limited to individual components alone.

An effective amount of therapeutic will decrease the symptoms typically by at least about 10%; usually by at least about 20%; preferably at least about 30%; or more preferably at least about 50%. The present invention provides reagents which will find use in therapeutic applications as described elsewhere herein, e.g., in the general description for treating disorders associated with the indications described above. Berkow (ed.) The Merck Manual of Diagnosis and Therapy, Merck & Co., Rahway, N.J.; Braunwald, et al. (eds.) (2001) Harrison's Principles of Internal Medicine, McGraw-Hill, NY; Gilman, et al. (eds.) (1990) Goodman and Gilman's: The Pharmacological Bases of Therapeutics, 8th ed., Pergamon Press; Remington's Pharmaceutical Sciences, 17th ed. (1990), Mack Publishing Co., Easton, Penn; Langer (1990) Science 249:1527-1533; Merck Index, Merck & Co., Rahway, New Jersey; and Physician's Desk Reference (PDR); Cotran, et al. (eds), supra; and Dale and Federman (eds.) (2000) Scientific American Medicine, Healtheon/WebMD, New York, NY.

### EXAMPLES

I. General Methods.
Some of the standard methods are described or referenced, e.g., in Maniatis, et al. (1982) Molecular Cloning, A Laboratory Manual, Cold Spring Harbor Press, Cold Spring Harbor, NY; Sambrook, et al. (1989) Molecular Cloning: A Laboratory Manual, (2d ed.), vols. 1-3, CSH Press, NY; Ausubel, et al., Biology, Greene Publishing Associates, Brooklyn, NY; or Ausubel, et al. (1987 and Supplements) Current Protocols in Molecular Biology, Greene/Wiley, New York. Methods for protein purification include such methods as ammonium sulfate precipitation, column chromatography, electrophoresis, centrifugation, crystallization, and others. See, e.g., Ausubel, et al. (1987 and periodic supplements); Deutscher (1990) "Guide to Protein Purification" in Meth. Enzymol., vol. 182, and other volumes in this series; and manufacturer's literature on use of protein purification products, e.g., Pharmacia, Piscataway, N.J., or Bio-Rad, Richmond, CA. Combination with recombinant techniques allow fusion to appropriate segments, e.g., to a FLAG sequence or an equivalent which can be fused via a protease-removable sequence. See, e.g., Hochuli (1990) "Purification of Recombinant Proteins with Metal Chelate Absorbent" in Setlow (ed.) Genetic Engineering, Principle and Methods 12:87-98, Plenum Press, N.Y.; and Crowe, et al. (1992) OIAexpress: The High Level Expression & Protein Purification System QIAGEN, Inc., Chatsworth, CA.
Software packages for determining, e.g., antigenic fragments, signal and leader sequences, protein folding, and functional domains, are available. See, e.g., Vector NTI® Suite (Informax, Inc., Bethesda, MD); GCG Wisconsin Package (Accelrys, Inc., San Diego, CA), and DeCyphe® (TimeLogic Corp., Crystal Bay, Nevada); Menne, et al. (2000) Bioinformatics 16:741-742. Public sequence databases were also used, e.g., from GenBank and others.
II. TSLP/IL-50 (SEQ ID NO:1) Activation of CD11c⁺DCs.
CD11c⁺ DC were purified from adult blood buffy coats of healthy volunteer blood donors (Stanford Medical School Blood Center, Stanford, CA) after separation of PBMC by Ficoll centrifugation and negative depletion of cells expressing CD3, CD14, CD19, CD56, and glycophorin A using magnetic beads (Dynal, Oslo, Norway). Depleted cells were further stained with anti-CD4-TC (Caltag, Burlingame, CA), anti-CD11c-PE and anti-CD3, CD 14, CD 16-FITC (Becton Dickinson, Franklin Lakes, NJ). CD11c⁺ CD4⁺ T cells were isolated using a Vantage FACsorter® (Becton Dickinson, Franklin Lakes, NJ) to reach >99% purity.
CD11c⁺ DC were cultured immediately after sorting in RPMI containing 10% FCS, 1% pyruvate, 1% HEPES, and penicillin/streptomycin. Cells were seeded at 0.5x106/ml in flat-bottom 96-well plates in the presence of TSLP/IL-50 (SEQ ID NO:1 ) (15 ng/ml), IL-7 (50 ng/ml), LPS (1mg/ml.), CD40-ligand-transfected L-fibroblasts (2.5 x 104/well) or culture medium alone. After 24 hours of culture, DC were harvested and re-suspended in an EDTA-containing medium to dissociate the clusters. Viable DC were first counted using trypan blue exclusion of dead cells.
Remaining cells were stained with a variety of mouse anti-human FITC-conjugated monoclonal antibodies (mAb) including anti-HLA-DR (Becton Dickinson, Franklin Lakes, NJ), anti-CD40, CD80 and CD86 (all from Pharmingen, San Diego, CA) or an IgG1 isotype control (Becton Dickinson, Franklin Lakes, NJ), and were analyzed with a FACScan® flow cytometer (Becton Dickinson, Franklin Lakes, NJ). Dead cells were excluded based on side and forward scatter characteristics. For apoptosis detection, cells were stained for 5-10 min with Annexin V-FITC (Promega, Madison, WI) and analyzed on a FACScan® flow cytometer (Becton Dickinson, Franklin Lakes, NJ) without dead cell exclusion. TSLP/IL-50 (SEQ ID NO:1), IL-7, CD40-ligand and LPS all upregulated surface HLA-DR, CD40, CD80, CD86 and CD83 on DCs when compared with medium alone. hTSLP/IL-50 (SEQ ID NO:1) was at least twice as potent as IL-7 in upregulating these markers. Interestingly, whereas TSLP/IL-50 (SEQ ID NO:1) induced the highest levels of CD40 and CD80 expression on DCs, CD40-ligand induced higher levels of HLA-DR and CD83. The ability of TSLP/IL-50 (SEQ ID NO:1) to upregulate HLA-DR and co-stimulatory molecules was blocked by neutralizing monoclonal antibodies specific for human TSLP/IL-50 (SEQ ID NO:1), indicating that the observed effects of TSLP/IL-50 (SEQ ID NO:1) on CD11c⁺ DCs were specific. Like CD40L, TSLP/IL-50 (SEQ ID NO:1) not only activated DCs, but also maintained the survival of DCs in 24h cultures as shown by Anexin V staining and cell counts. Morphologically, both TSLP/IL-50 stimulated-DCs and CD40L-DCs display long dendrites, and express HLA-DR and dendritic cell-lysosome-associated membrane glycoprotein (DC-LAMP), when compared with medium-DCs or IL-7-DCs.
DC-LAMP is a DC activation marker. DC-LAMP is rapidly induced by TNFalpha, LPS, or CD40L, and may be used for antigen presentation (Saint-Vis, et al. (1998) Immunity 9:325-336).
III. Priming of Naïve CD4 T cells.
CD11c⁺ DC were harvested after 24h of culture in different conditions, washed twice to remove any cytokine and co-cultured with 5x10⁴ freshly purified allogeneic naïve CD4⁺ T cells in round-bottom 96-well culture plates. Co-cultures were carried out in triplicate at increasing DC/T cell ratios. DC and T cells alone were used as controls. After 5 days, cells were pulsed with 1 mCi ³H-thymidine (Amersham Biosciences Corp., Piscataway, NJ) for 16 hours before harvesting and counting of radioactivity.
Most strikingly, TSLP/IL-50 stimulated-DCs induced the strongest naive CD4 T cell proliferation in allogeneic mixed lymphocyte reaction, when compared to CD40L-DCs, LPS-DCs or IL-7-DCs. At a ratio of 1 DC per 150 T cells, TSLP/IL-50 (SEQ ID NO:1)-activated DCs still induced a very strong allogeneic naïve CD4 T cell proliferation, which was about 10 times stronger than that induced by CD40L-DCs. After 6 days of culture, TSLP/IL-50 stimulated-DCs induced a 2.5 to 10-fold increase in total T cell numbers, more than that induced by CD40L-DCs, LPS-DC or IL-7-DC. Therefore, human TSLP/IL-50 (SEQ ID NO:1) represents one of the most potent DC activation factors and TSLP/IL-50 stimulated-DC induce the most impressive allogeneic naive CD4 T cell proliferation and expansion.
IV. Cytokine and Chemokine Expression of DC primed Naïve T Cells. T cells were harvested at day 6 of the co-culture, washed twice and re-stimulated with PMA and ionomycine in flat bottom 96- or 48-well plates at a concentration of 1x10⁶/ml. After 2.5h, Brefeldin A was added at 10mg/ml. After 5h, cells were harvested, fixed with 2% formaldehyde, permeabilized with 10% saponin and stained with PE-conjugated mAbs to IL-4, IL-5, IL-10, IL-13 and TNFalpha and FITC-conjugated mAb to IFN-gamma (all from Pharmingen, San Diego, CA). Stained cells were analyzed on a FACScan^{®} flow cytometer (Becton Dickinson, Franklin Lakes, NJ).
Previous studies have shown that most DC activation signals such as CD40L and LPS induce DCs to produce pro-inflammatory cytokines (IL-lalpha/beta, IL-6 and IL-12) and to prime naïve CD4 T cell differentiation towards TH1 (Guermonprez, et al. (2002) Annu. Rev. Immunol. 20:621-667; Banchereau, et al. (2000) Annu. Rev. Immunol. 18:767-811). To investigate the effects of TSLP/IL-50 (SEQ ID NO:1) on DC cytokine expression, we first performed a global quantitative mRNA screening of 11 different cytokines (IL-lalpha, IL-lbeta, IL-4, IL-6, IL-10, IL-12p35, IL-12p40, IL-13, IL-18, IL-23p19 and TNFalpha) and 12 different chemokines (TARC, DCCK1, MDC, MCP1, MCP2, MCP3alpha, MCP4, eotaxin, MIP3, MIG, Rantes and IL-8). Surprisingly, unlike CD40L-DCs, TSLP/IL-50-treated DCs did not produce mRNA for all the pro-inflammatory cytokines tested, but produced high levels of mRNA for the chemokines TARC and MDC. ELISA analyses confirmed at the protein level that TLSP-activated DCs did not produce detectable amounts of pro-inflammatory cytokines IL-lbeta, IL-6, IL-12p70 and TNFalpha, but high levels of the chemokines TARC and MDC (Reche, et al. (2001) J. Immunol. 167:336-343). TARC and MDC preferentially attract CCR4-expressing TH2 cells.
Next, the capacity of hTSLP/IL-50 stimulated-DC to polarize naïve CD4 T cells was compared to DCs respectively cultured with medium, IL-7, CD40L or LPS. Human CD4⁺CD45RA⁺ naïve T cells purified from adult peripheral blood were co-cultured with DCs at a 1/5 ratio for 6 days, washed to remove all cytokines, re-stimulated 24 hours with anti-CD3 and anti-CD28, and cytokine production was measured in the culture supernatant by ELISA. Strikingly, TSLP/IL-50 stimulated-DCs induce naive CD4 T cells to produce the highest levels of TH2 cytokines IL-4, IL-5 and IL-13, together with the pro-inflammatory cytokine TNFalpha. TSLP/IL-50 stimulated-DCs induce naive CD4+ T cells to produce the lowest levels of anti-inflammatory cytokine IL-10 and TH1 cytokine IFN-gamma, when compared with DCs cultured with medium alone, or other activators. The ability of TSLP/IL-50 stimulated-DCs to induce naive CD4 T cells to produce high IL-4, IL-13 and TNFalpha and low IFN-gamma and IL-10 was confirmed by intracellular cytokine staining. Therefore, TSLP/IL-50 (SEQ ID NO:1)-DCs induced naïve CD4 T cells to produce a very unique set of cytokines, which is distinct from a TH1 profile (IFN-gamma) or a classical TH2 profile (IL-4, IL-5 and IL-10). TSLP/IL-50 stimulated-DCactivated CD4 T cells produced the highest levels of TNFalpha, one of the most potent pro-inflammatory cytokines, when compared with CD4 T cells activated respectively by medium-DC, IL-7-DC, CD40L-DC or LPS-DC. On the contrary, TSLP/IL-50 stimulated-DC appeared to inhibit CD4+ T cells to produce IL-10, a potent anti-inflammatory cytokine (see, e.g., Moore, et al. (2001) Annu Rev Immunol 19:683-765) as well as IFN-gamma, a TH1 cytokine which can cross inhibit TH2 response (Abbas, et al. (1996) Nature 383:787-793). Therefore, TSLP/IL-50 stimulated-DCs induce robust TH2 allergic inflammation by promoting naïve CD4⁺ T cells to produce IL-4, IL-5 and IL-13, in the presence of a strong pro-inflammatory cytokine TNFalpha, and in the absence of two physiologic inhibitors of Th2 inflammation, IL-10 and IFN-gamma. In addition, TSLP/IL-50 stimulated-DCs further enhance TH2-mediated inflammation by producing chemokines such as TARC and MDC, which preferentially recruit TH2 cells into the original inflamed tissues (see, e.g., Imai et al. (1999) Int. Immunol. 11:81-88; Andrew et al. (1998) J. Immunol. 161:5027-5038; Andrew et al. (2001) J. Immunol. 166:103-111; Vestergaard et al. (2000) J. Invest. Dermatol. 115:640-646; and Vestergaard et al. (1999) J. Clin. Invest. 104:1097-1105).
V. Expression of TSLP/IL-50.
A. Stromal Cells.
To further understand the biology and pathophysiology of TSLP/IL-50 (SEQ ID NO:1), the expression of TSLP/IL-50 (SEQ ID NO:1) mRNA was analyzed by real time quantitative PCR (Taqman®) in a panel of cDNA libraries from different primary cells or cell lines, and a panels of FACS-sorted primary cells (cell purity over 99%). TSLP/IL-50 (SEQ ID NO:1) expression was not found in most hematopoietic cell types, including B cells, T cells, NK cells, granulocytes, macrophages, monocyte subsets, and DC subsets. Interestingly, mast cells activated by monoclonal antibodies which cross-link high affinity IgE receptors express very high levels of hTSLP/IL-50 (SEQ ID NO:1). hTSLP/IL-50 (SEQ ID NO:1) was found to be highly expressed by cultured human primary stromal cells such as skin keratinocytes, epithelial cells, smooth muscle cells, and lung fibroblasts. Bronchial smooth muscle cells and skin keratinocytes activated respectively by IL-4, IL-13 and TNFalpha, or TNFalpha and IL-lbeta appear to express higher hTSLP/IL-50 (SEQ ID NO:1), when compared with medium only controls. TSLP/IL-50 (SEQ ID NO:1) expression was not found in endothelial cells. Therefore, hTSLP/IL-50 (SEQ ID NO:1) mRNA is mainly expressed by most stromal cell types and mast cells, but not by most hematopoietic cell types and endothelial cells.
Primary cells consisting of bronchial smooth muscle cells (BSMC), normal human lung fibroblasts (NHLF), normal human epidermal keratinocytes (NHEK), and lung fibroblast cell line (MRC5) were seeded at 0.5 X 10⁶ cells in six well tissue culture plates. Cytokines or combinations of cytokines were added at the indicated concentrations followed by incubation for 8 h at 37°C.
Expression of TSLP/IL50 mRNA by human bronchial smooth muscle cells (BSMC) exposed to various cytokines was assessed by Taqman® and ELISA following treatment of cells with various cytokines, as described in Soumelis, et al. (2002) Nature Immunol. 3:673-680; Reche, et al. (2001) J. Immunol. 167:336-343). Expression of mRNA levels was adjusted as units relative to expression of 18s RNA. Cells were treated with medium only, IL-lalpha, IL-lbeta, TNFalpha, or the combination of IL-lbeta and TNFalpha, at concentrations of 0, 0.001, 0.01, 0.1, 1.0, or 10 ng/ml, in separate incubation mixtures. ELISA results showed similar patterns where the combination of IL-lbeta and TNFalpha elicited the highest expression of TSLP/IL-50 from BSMC. Taqman and ELISA results are summarized in Table 1A.
IL-8 production from stromal cells was used as a control (Table 1B). A comparison across the four cell lines tested revealed differences in trends in TSLP-IL-50 expression and IL-8 expression, indicating that the mechanisms leading to TSLP/IL-50 and IL-8 expression are not identical.

**Table 1A. Expression of TSLP/IL-50 mRNA and protein from stromal cells. TREATMENT (ND; not determined)**

| Cells | Technique | IL-1alpha | IL-1beta | TNFalpha | IL-lbeta + TNFalpha |
|---|---|---|---|---|---|
| BSMC | Taqman | 321 x 10⁻⁷ | 418 x 10⁻⁷ | 858 x 10⁻⁷ | 927 x 10⁻⁷ |
| | ELISA (ng/ml) | 0.73 | 0.16 | 0.13 | 0.90 |
| NHLF | Taqman | 18 x 10⁻⁷ | ND | ND | 21 x 10⁻⁷ |
| | ELISA (ng/ml) | 0.06 | ND | ND | 0.10 |
| NHEK | Taqman | 12 x 10⁻⁷ | ND | ND | 10 x 10⁻⁷ |
| | ELISA (ng/ml) | 0.012 | ND | ND | 0.017 |
| MRC5 | Taqman | 54 x 10⁻⁷ | ND | ND | 130 x 10⁻⁷ |
| | ELISA (ng/ml) | 0.17 | ND | ND | 0.09 |

**Table 1B. Expression of IL-8 mRNA and protein from stromal cells. TREATMENT (ND; not determined)**

| Cells | Technique | IL-1alpha | IL-1beta | TNFalpha | IL-1beta + TNFalpha |
|---|---|---|---|---|---|
| BSMC | Taqman | 730 x 10⁻⁴ | 292 x 10⁻⁴ | 118 x 10⁻⁴ | 1331 x 10⁴ |
| | ELISA (ng/ml) | 16 | 32 | 14 | 31 |
| NHLF | Taqman | 340 x 10⁻⁴ | ND | ND | 345 x 10⁻⁴ |
| | ELISA (ng/ml) | 41 | ND | ND | 36 |
| NHEK | Taqman | 2.6 x 10⁴ | ND | ND | 5.2 x 10⁴ |
| | ELISA (ng/ml) | 0 | ND | ND | 1.1 |
| MRC5 | Taqman | 156 x 10⁻⁴ | ND | ND | 411 x 10⁴ |
| | ELISA (ng/ml) | 104 | ND | ND | 36 |

Separate tests demonstrated that treatment with IL-13 (25 ng/ml; 8 h) stimulated normal human lung fibroblasts (NHLF) and normal human dermal fibroblasts to express TSLP/IL-50, while treatment with IL-17 (25 ng/ml; 8 h) provoked BSMC cells and normal human dermal fibroblasts to express TSLP/IL-50. Expression in response to IL-13 or IL-17 was not detected from, e.g., normal human epidermal keratinocytes. B. Inflamed Tonsils.
To determine whether human inflamed tissues, such as tonsils, express hTSLP/IL-50 (SEQ ID NO:1) protein, immunohistology was investigated. Samples were stained using mAb 6NE0112F3, which specifically recognizes hTSLP/IL-50. Human tonsils contain crypt epithelium, which lines the crypts and which frequently harbor viruses and bacteria and represents the sites of antigen-entry and constitutive inflammation, and squamous epithelium, which lines the tonsil surface. Among all five different tonsillar samples, hTSLP/IL-50 (SEQ ID NO:1) was found to be constitutively expressed by crypt epithelial cells, which are in close contact with DC-LAMP positive lymphocytes and activated dendritic cells. Interestingly in all tonsil samples, only a few small foci of hTSLP/IL-50 (SEQ ID NO:1) expression were found within the apical part of the squamous epithelium. The expression of TSLP/IL-50 (SEQ ID NO:1) was associated with the infiltration of DC-LAMP positive activated DCs and the concurrent loss of langerin-positive Langerhans cells within the squamous epithelium. hTSLP/IL-50 (SEQ ID NO:1) contributes to the constitutive inflammation within the crypt epithelium and the sporadic inflammation within the squamous epithelium. C. Keratinocytes in Atopic Dermatitis.
To investigate whether hTSLP/IL-50 (SEQ ID NO:1) expression was associated with Th2-type allergic inflammation in vivo, hTSLP/IL-50 protein expression was analyzed in skin lesions, including atopic dermatitis (a TH2 mediated allergic disease), nickel-induced contact dermatitis (a IFN-gamma-producing CD8⁺ T cells mediated allergic disease) and disseminated lupus erythematosus (a TH1-mediated disease). While hTSLP/IL-50 was not detectable in normal skin, and non-lesional skin of atopic dermatitis, high expression of hTSLP/IL-50 was found in keratinocytes of acute (4 patients) and chronic atopic dermatitis (6 patients). The expression of hTSLP/IL-50 was found mainly in keratinocytes of the apical layers of the epidermis, ranging from small foci to the whole apical areas in both acute and chronic atopic dermatitis. hTSLP/IL-50 was not found in skin lesions from nickel-induced allergy contact dermatitis and disseminated lupus erythematosus.
VI. Langerhans cell migration and activation.
To investigate whether hTSLP/IL-50 (SEQ ID NO:1) expression in atopic dermatitis associates with DC activation, hTSLP/IL-50 was stained together with either langerin (a Langerhans cell marker), or DC-LAMP (a DC activation marker), by double immunohistology. In normal skin, or non-lesional skin of atopic dermatitis, many langerin-positive Langerhans cells were found only within the epidermis, but not within the dermis, and no DC-LAMP⁺ DCs were found in either the epidermis or dermis. The strong hTSLP/IL-50 expression in atopic dermatitis was associated with disappearance of langerin-positive Langerhans cells within the epidermis, and concurrent appearance of many DC-LAMP⁺ DCs within the dermis. Many of the DC-LAMP⁺ DCs within the dermis express langerin, showing that epidermal Langerhans cells are activated and migrate into the dermis. Thus, hTSLP/IL-50 expression by keratinocytes of atopic dermatitis contribute directly to the activation of Langerhans cells, which migrate into the draining lymph nodes and prime allergen-specific TH2 responses.
VII. Expression of TSLP/IL-50 by human cells.
Expression of TSLP/IL-50 was determined by Taqman®, as described previously. The relative expression of TSLP/IL-50 in the indicated cells was: cultured lung fibroblasts (++++); cultured bronchial smooth muscle (++++); prostate stromal cells (++); mammary stromal cells (+); mammary epithelial cells (+); hepatofibroblasts (+); skin keratinocytes (+).
Expression of TSLP/IL-50 was also determined by histological methods. Thymic epithelial cells were stained with tagged anti-TSLP/IL-50 antibody, as described in Soumelis, et al., supra. TSLP/IL-50 was not detected in keratinocytes from normal skin and in non-lesional skin sections from atopic dermatitis, while high expression was found in keratinocytes of acute and chronic atopic dermatitis. In normal skin, expression was not found in sweat glands, eccrine glands, and hair follicles. TSLP/IL-50 expression was also expressed by thymic epithelial cells (Hassal corpuscules), as determined by histology.
VIII. Allogeneic and autologous hTSLP/IL-50-treated DC both induce proliferation of naive CD4+ T cells.
Naive CD4+ T cells were exposed to allogeneic dendritic cells prepared under one of five different test conditions, followed by assessment of proliferation of the T cells. The five conditions are described in Table 2. Proliferation was determined by 3H-thymidine incorporation assays. In allogeneic reactions, DC treated with TSLP/IL-50 (SEQ ID NO:1) produced the greatest increased in T cell proliferation, while DC treated with other agents resulted in lesser or much lesser T cell proliferation.
Autologous cell interactions, where CD11c+ dendritic cells and CD4+ T cells were from the same human donor, were tested (Table 2). Again, use of DC treated with TSLP/IL-50 (SEQ ID NO:1) produced the greatest increase in T cell proliferation, while other preparations of DC produced lesser levels of T cell proliferation.

**Table 2.**

| Fold-increase in proliferation of CD4+ T cells with allogenic and autologous reactions. | | |
|---|---|---|
| TEST CONDITION | Increase in CD4+ T cell number | |
| | ALLOGENIC | AUTOLOGOUS |
| 1. TSLP/50-treated dendritic cells (DC). | 8.5-fold | 5.5-fold |
| 2. Lipopolysaccharide (LPS). | 3.6 | 1.0 |
| 3. CD40L-treated DC. | 3.3 | 1.8 |
| 4. IL-7 treated DC. | 1.7 | 1.3 |
| 5. Medium-treated DC. | 1.0 | 1.0 |

IX. TSLP/IL-50 (SEQ ID NO:1)-treated DC stimulates proliferation of naïve CD4+ T cells. TSLP/IL-50-activated DC were mixed with autologous naive CD4⁺ T cells followed by an assessment of the profile of subspecies of T cell receptor in the pool of expanded, proliferating T cells. The subspecies of T cell receptor assayed for were TCRVβ1, TCRVβ2, TCRVβ3, TCRβ5, TCRVβ8, TCRVβ4, TCRVβ7, TCRVβ22, and TCRVβ23. Three types of control incubations were used: ( 1 ) Untreated T cells; (2) T cells treated with IL-7; and ( 3 ) T cells treated with Streptococcus endotoxin B-activated DC. The untreated control population of T cells contained subspecies of T cell receptor as indicated: TCRVβ1 (about 3%), TCRVβ2 (about 8%), TCRVβ3 (about 6%), TCRβ5 (about 2%), TCRVβ8 (about 4%), TCRVβ4 (about 2.5%), TCRVβ17 (about 7%), TCRVβ22 (about 2.5%), and TCRVβ23 (about 0.2%). Naïve CD4+ T cells treated with TSLP/IL-50 (SEQ ID NO:1)-activated DC (experimental), followed by incubation to allow proliferation of the T cells, exhibited a profile of T cell receptor subspecies that was very similar to that found with the non-cultured T cells. The similar profiles found in the non-cultured T cells and in T cells treated with TSLP/IL-50-activated DC demonstrated that polyclonal expansion of the T cells had occurred. Control incubation with IL-7 (no DC) also resulted in polyclonal expansion of T cells, while control incubation with endotoxin-treated DC resulted in the selected expansion of T cells bearing TCRVβ3 and TCRVβ17.
X. Expansion of T cells mediated by TSLP/IL-50 is long lasting. TSLP/IL-50-treated DC were incubated with autologous naive CD4⁺ T cells, followed by assessment of cell number at t = 0, 6, 9, 12, 15, 18, and 21 days. With exposure to TSLP/IL-50 (SEQ ID NO:1)-activated DC, T cell number increased by 10-fold at day 15, followed by a drop in cell number, at later time points. Control incubations used naïve CD4+ T cells exposed to IL-7-activated DC, to LPS-activated DC, to poly I:C-activated DC, to CD40L-activated DC, and to medium-treated DC. Essentially all control incubations resulted in little or no increase in T cell number, though a 3-fold increase in T cell number was found at day 6 with LPS-activated DCs.
XI. Alteration of T cell phenotype.
The phenotype of naïve CD4+ T cells before and after treatment with TSLP/IL-50 (SEQ ID NO:1)-activated DC was determined. Phenotype was assessed by measuring the following markers on the T cells: CD45RA; CD45RO; CD25; CD62L; and CCR7.
Naive T cells have the phenotype CD45RA⁺, CD45RO⁻, CD25-, CD62L⁺, and CCR7⁺; central memory T cells have the phenotype CD45RA-, CD45RO+, CD25+/-, CD62L⁺, and CCR7⁺; and effector memory T cells have the phenotype CD45RA-, CD45RO+, CD25^{+/-}, CD62L^{+/-}, and CCR7⁻. CD4⁺T cells activated with TSLP/IL-50 (SEQ ID NO:1)-treated DC had the phenotype of central memory T cells.
Control incubations revealed that treating naive CD4⁺ T cells with IL-7, but no DC, resulted in no change in phenotype. Treating naïve CD4⁺ T cells with DC plus IL-2 resulted in T cells of the phenotype CD45RA⁻, CD45RO⁺, CD25⁺, CD62L⁺, and CCR7^{+/-}.
Naive CD4⁺ T cells were exposed to autologous TSLP/IL-50 (SEQ ID NO:1)-activated DC, followed by expansion of the T cells. The population of expanded T cells was tested for secretion of the following cytokines: IL-2, IFN-gamma, IL-10, IL-4, IL-5, and IL-13. Thus, the results demonstrated high secretion of IL-2, low secretion of IL-5 and IL-13, and little to no secretion of IFN-gamma, IL-10, and IL-4. The expanded CD4+ T cells lack immediate effector function.
Naive CD4⁺ T cells were exposed to allogeneic TSLP/IL-50 (SEQ ID NO:1)-activated DC, followed by expansion of the T cells and secretion of the above cytokines was assessed. The results demonstrated high secretion of IL-2, IL-4, IL-5, and IL-13, and low secretion of IFN-gamma, indicating that the allogeneic expanded CD4+ T cells had a Th2-type cytokine profile. Expression of the above-identified cytokines can be used in the detection of autologous or allogeneic reactions or pathological conditions involving TSLP/IL-50-activated APCs.
Use of T cells expanded by autologous reaction, rather than allogeneic reaction, might be preferred therapeutically where immediate effector response, e.g., inflammatory response, is not desired.
Naive CD4⁺ T cells exposed to autologous TSLP/IL-50 (SEQ ID NO:1)-activated DC and are treated with anti-CD3 plus anti-CD28, along with anti-IL-4 plus IL-12 (reagents known to promote a TH1 profile), the result is T cells secreting large amounts of IFN-gamma, but low levels of IL-2, IL-4, IL-10, and IL-13, i.e., effector cells with a TH1 profile.
Thus, naive CD4⁺ T cells exposed to autologous TSLP/IL-50-activated DC can lack immediate effector function, but can be stimulated to differentiate into effector cells by secondary stimulation. These results indicate that autologous TSLP/IL-50-activated DC are able to mount an antigen-dependent response in vivo, e.g., in response to a pathogen.
Naïve CD4⁺ T cells were treated for seven days with autologous TSLP/IL-50-activated DC, followed by washing of the cells. The cells were then titrated with anti-CD3 with constant levels of anti-CD28, in order to cause TCR signaling. As a control, naïve CD4⁺ T cells were also titrated with anti-CD3 with constant levels of anti-CD28. Separate incubation mixtures contained anti-CD3 at 0.0001, 0.0003, 0.001, 0.003, 0.01, 0.03, 0.1, 0.3, 1.0, 3.0, or 10.0 microgram/ml, while all incubations contained anti-CD28 at a constant level of 1.0 microgram/ml. Proliferation of the T cells was measured by 3H-thymidine incorporation. The results demonstrated that the naive CD4⁺ T cells were maximally stimulated to proliferate with anti-CD28 at about 3.0 microgram/ml, with little or no stimulation found with lower levels of anti-CD28. In contrast, naïve CD4⁺ T cells treated with autologous TSLP/IL-50-activated DC were maximally stimulated to proliferate at much lower levels of anti-CD28, i.e., at about 0.1 microgram/ml. Thus, CD4⁺ T cells expanded with autologous TSLP/IL-50-activated DC have a reduced threshold of activation.
XII. TSLP/IL-50 (SEQ ID NO:1)-activated DC induces proliferation of various CD4⁺ T cells.
TSLP/IL-50 (SEQ ID NO:1)-activated DC were incubated with autologous: ( 1 ) Naive CD4⁺ T cells; ( 2 ) Central memory CD4⁺ T cells; or ( 3 ) Autologous effector memory CD4⁺ T cells; with assessment of T cell proliferation by 3H-thymidine incorporation. Separate incubations were conducted with DC/T cells at a ratio of 1:1; 1:2; 1:4; 1:8; 1:16; 1:32; 1:64. Control incubations included T cells only and medium only. With assessment of proliferation, maximal proliferation of each of the three populations of T cells was found to occur with DC/T cells at the 1:1 ratio. Proliferation of naïve T cells was generally 1.2 to 1.8-fold greater than proliferation of central memory T cells, while proliferation of central memory T cells was generally about 2-fold greater than that of effector T cells. Controls incubated with each of the three types of T cells resulted in little or no induction of T cell proliferation.
XIII. Psoriasis and TSLP/IL-50 expression. Samples of normal human skin and psoriatic skin from 10 different subjects each were analyzed by histological methods. Staining was performed with anti-TSLP/IL-50 antibody or with control IgG2a antibody (cat. no. M68178; Pharmingen Inc., San Diego, CA), both tagged with peroxidase AEC (Vector Laboratories, Inc., Burlingame, CA). Anti-TSLP/IL-50 antibodies from two different clones were used, where the results from both sources of anti-TSLP/IL-50 were consistent with each other. Staining was assessed in keratinocytes, hair follicles, and eccrine glands. Keratinocyte staining in all ten normal subjects was negative. Hair follicle and eccrine gland staining in the ten normal subjects ranged from negative or low. Keratinocyte staining from the ten psoriatic subjects was high, where hair follicle and eccrine gland staining was comparatively lower. The results demonstrated a significant association between TSLP/IL-50 expression and psoriasis.

### SEQUENCE IDENTIFIERS

SEQ ID NO:1 is human thymic stromal lymphopoietin (hTSLP/IL-50).

SEQ ID NO:2 is IL-7R-alpha chain.

SEQ ID NO:3 is TSLP receptor (TSLPR).

All citations herein are incorporated herein by reference to the same extent as if each individual publication, patent application, or patent was specifically and individually indicated to be incorporated by reference including all figures and drawings.

Many modifications and variations of this invention, as will be apparent to one of ordinary skill in the art can be made to adapt to a particular situation, material, composition of matter, process, process step or steps, to preserve the objective, spirit and scope of the invention. All such modifications are intended to be within the scope of the claims appended hereto without departing from the spirit and scope of the invention. The specific embodiments described herein are offered by way of example only, and the invention is to be limited by the terms of the appended claims, along with the full scope of equivalents to which such claims are entitled; and the invention is not to be limited by the specific embodiments that have been presented herein by way of example.

## Claims

1. A method of modulating antigen presenting cell (APC) priming of a T cell comprising contacting the APC with:
a) an agonist of TSLP/IL-50 (SEQ ID NO:1) or TSLP/IL-50 receptor (TSLP/IL-50R) (SEQ ID NOs:2, 3); or
b) an antagonist of TSLP/IL-50 (SEQ ID NO:1) or TSLP/IL-50R (SEQ ID NOs:2, 3).

2. The method of Claim 1, wherein the T cell is a naïve CD4⁺ T cell, a central memory T cell, or an effector memory T cell.

3. The method of Claim 1, wherein the APC is a CD11c⁺ dendritic cell (DC).

4. The method of Claim 1, wherein the priming stimulates the proliferation of the T cell.

5. The method of Claim 4, wherein the proliferation is polyclonal.

6. The method of Claim 1, wherein the interaction between the APC and the T cell is autologous or allogeneic.

7. The method of Claim 6, wherein the interaction is autologous and yields a central memory T cell phenotype.

8. The method of Claim 1, wherein the agonist or antagonist comprises:
a) a humanized antibody;
b) a monoclonal antibody;
c) a polyclonal antibody;
d) an Fab fragment;
e) an F(ab')₂ fragment; or
f) a peptide mimetic of an antibody.

9. The method of Claim 1, wherein the agonist comprises TSLP/IL-50 (SEQ ID NO:1), or an antigenic fragment thereof.

10. A method of treating a subject suffering from an immune disorder comprising treating with or administering an effective amount of:
a) an agonist of TSLP/IL-50 (SEQ ID NO:1) or TSLP/IL-50 R (SEQ ID NOs:2,3); or
b) an antagonist of TSLP/IL-50 (SEQ ID NO:1) or TSLP/IL-50R (SEQ ID NOs:2, 3).

11. The method of Claim 10, wherein the immune disorder is an inflammatory condition and the administration comprises an effective amount of an antagonist of TSLP/IL-50 (SEQ ID NO:1) or TSLP/IL-50R (SEQ ID NOs:2, 3).

12. The method of Claim 11, wherein the immune disorder is psoriasis, psoriatic arthritis, or pulmonary inflammatory response.

13. The method of Claim 12, wherein the pulmonary inflammatory disease is asthma or chronic obstructive pulmonary disorder (COPD).

14. The method of Claim 10, wherein the immune disorder is immunodeficiency and the administration comprises an effective amount of an agonist of TSLP/IL50 (SEQ ID NO:1).

15. The method of Claim 14, wherein the immunodeficiency is a result of cytoablation or viral infection causing immunosuppression.

16. The method of Claim 10, wherein the administration comprises ex vivo treatment of autologous or allogeneic antigen presenting cells (APCs).

17. The method of Claim 16, wherein the administration comprises ex vivo treatment of APCs with an effective amount of an agonist of TSLP/IL50 (SEQ ID NO:1).

18. The method of Claim 10, wherein the agonist or antagonist comprises:
a) a humanized antibody;
b) a monoclonal antibody;
c) a polyclonal antibody;
d) an Fab fragment;
e) an F(ab')₂ fragment; or
f) a peptide mimetic of an antibody.

19. The method of Claim 10, wherein the agonist comprises TSLP/IL-50 (SEQ ID NO:1), or an antigenic fragment thereof.

20. A method of inducing production of IL-4, IL-5, and IL-13 by a T cell comprising:
a) contacting an APC with an agonist of TSLP/IL-50 or TSLP/IL-50 receptor; and
b) priming the T cell with the APC.

21. A method of modulating TH2 response in a subject comprising administration of:
a) an agonist of TSLP/IL-50 (SEQ ID NO:1) or TSLP/IL-50 receptor (TSLP/IL-50R) (SEQ ID NOs:2, 3); or
b) an antagonist of TSLP/IL-50 (SEQ ID NO:1) or TSLP/IL-50R (SEQ ID NOs:2, 3).
